# EUROPEAN PATENT SPECIFICATION

(11) **EP 3 767 426 B1**
(45) Date of publication and mention of the grant of the patent: **11.12.2024**
(21) Application number: 18905568.4
(22) Date of filing: 12.02.2018
(51) Int. Cl.: G16H 40/40, G16H 40/63, G16H 40/67, G16H 10/40, A61B 5/022, A61B 5/00

(54) **IN-VITRO DIAGNOSIS DEVICE PARAMETER UPDATE METHOD AND DEVICE**
VERFAHREN UND VORRICHTUNG ZUR PARAMETERAKTUALISIERUNG EINER VORRICHTUNG ZUR IN-VITRO-DIAGNOSE
PROCÉDÉ ET DISPOSITIF DE MISE À JOUR DE PARAMÈTRES D'UN DISPOSITIF DE DIAGNOSTIC IN-VITRO

(43) Date of publication of application: 20.01.2021
(73) Proprietor: Shenzhen IncreCare Biotech Co., Ltd, Shenzhen, Guangdong 518000 (CN)
(72) Inventor: ZHANG, Zhen, Shenzhen, Guangdong 518000 (CN); LIU, Qilin, Shenzhen, Guangdong 518000 (CN); YU, Huaibo, Shenzhen, Guangdong 518000 (CN); YAO, Yanyi, Shenzhen, Guangdong 518000 (CN); ZHENG, Wenyang, Shenzhen, Guangdong 518000 (CN); HE, Taiyun, Shenzhen, Guangdong 518000 (CN)
(74) Representative: Novagraaf Technologies
(86) International application number: PCT/CN2018/076541
(87) International publication number: WO 2019/153354

(56) References cited:
- WO-A2-2013/020045
- CN-A- 102 023 100
- CN-A- 104 766 139
- CN-A- 105 468 787
- CN-A- 106 483 274
- CN-A- 106 844 536

## Description

### Technical Field

The present disclosure relates to the technical field of data processing of medical instrument and equipment, in particular to an *in-vitro* diagnostic device parameter updating method (method for updating parameters of an in-vitro diagnostic device) and an *in-vitro* diagnostic device parameter updating device (device for updating parameters of an in-vitro diagnostic device).

### Background Art

During use of an *in-vitro* diagnostic device, an assay parameter of an *in-vitro* diagnostic reagent o needs to be updated (for example, addition, deletion, change). An existing method for updating the assay parameter is as follows: first, a manufacturer using a manufacturer service platform to produce an assay parameter of an *in-vitro* diagnostic reagent according to an assay parameter identifier, then using a barcode, a mobile data storage device, a portable device or the like as a carrier for supplying and transferring data, finally, a user scanning the data carrier using a barcode scanner or other recognizers at a client end, and connecting a mobile storage device with the diagnostic device for data transmission or connecting a portable device with the diagnostic device for corresponding data transmission, so as to update the assay parameter of the in-vitro diagnostic reagent.

Document WO2013020045 discloses an in-vitro diagnostic device parameter updating method, which does not allow to update its own corresponding assay parameter value.

However, in such existing method for updating the assay parameter of the *in-vitro* diagnostic reagent, new data carrier is delivered to the client only when the assay parameter of the *in-vitro* diagnostic reagent needs to be updated after the assay parameter of the *in-vitro* diagnostic reagent is produced by the manufacturer service platform, then the data acquisition efficiency is low, and the data updating speed is slow.

### Summary

In view of this, it is necessary to provide an *in-vitro* diagnostic device parameter updating method and an *in-vitro* diagnostic device parameter updating device, which can realize efficient assay parameter updating of an *in-vitro* diagnostic reagent.

An *in-vitro* diagnostic device parameter updating method, characterized by comprising following steps of:
identifying and collecting an assay parameter identifier, wherein the assay parameter identifier is used for determining an assay parameter;
sending a data processing request to a data service platform, wherein the data processing request carries the assay parameter identifier;
receiving an assay parameter value and a parameter identifier which are fed back by the data service platform; and
updating a corresponding local assay parameter value according to the fed-back assay parameter value and parameter identifier
wherein the assay parameter comprises reaction mode, sampling amount or incubation time associated with an *in-vitro* diagnostic reagent item,
wherein the data service platform comprises a manufacturer service platform and a third-party data service platform,
wherein the data processing request is sent to the third-party data service platform, then the third-party data service platform searches itself to find whether a corresponding assay parameter value exists or not, and
   - if the corresponding assay parameter value exists, the corresponding assay parameter value and parameter identifier are directly fed back to the *in-vitro* diagnostic device, and
   - if the corresponding assay parameter value does not exist, the data processing request from the *in-vitro* diagnostic device is sent to the manufacturer service platform, the manufacturer service platform determines the corresponding assay parameter value according to the assay parameter identifier, and feeds back corresponding assay parameter value and parameter identifier to the *in-vitro* diagnostic device through the third-party data service platform, or the manufacturer service platform itself feeds back the corresponding assay parameter value and parameter identifier to the *in-vitro* diagnostic device.

In the method, the step of identifying and collecting an assay parameter identifier may comprise:
collecting the assay parameter identifier by scanning an identity identification tag, wherein the identity identification tag comprises at least one of one-dimensional barcode, two-dimensional barcode and radio frequency tag.

In the method, the step of identifying and collecting an assay parameter identifier may comprise:
collecting the assay parameter identifier by receiving a parameter identifier identification code, wherein the parameter identifier identification code is an identification code input by a user.

In any one of these embodiments of the method, the step of sending a data processing request to a data service platform may comprise:
sending a data processing request to the data service platform through wireless communication, wherein the wireless communication comprises 3G, 4G, 5G, WiFi and bluetooth.

An *in-vitro* diagnostic device parameter updating device, characterized by comprising:
a collection module (200) configured to identify and collect an assay parameter identifier, wherein the assay parameter identifier is used for determining an assay parameter;
a sending module (300) configured to send a data processing request to a data service platform, wherein the data processing request carries the assay parameter identifier;
a reception module (400) configured to receive an assay parameter value and a parameter identifier which are fed back by the data service platform; and
an update module (500) configured to update a corresponding local assay parameter value according to the fed-back assay parameter value and parameter identifier,
wherein the assay parameter comprises reaction mode, sampling amount or incubation time associated with an *in-vitro* diagnostic reagent item,
wherein the data service platform comprises a manufacturer service platform and a third-party data service platform, and
wherein the data processing request is sent to the third-party data service platform, then the third-party data service platform searches itself to find whether a corresponding assay parameter value exists or not, and
   - if the corresponding assay parameter value exists, the corresponding assay parameter value and parameter identifier are directly fed back to the *in-vitro* diagnostic device, and
   - if the corresponding assay parameter value does not exist, the data processing request from the *in-vitro* diagnostic device is sent to the manufacturer service platform, the manufacturer service platform determines the corresponding assay parameter value according to the assay parameter identifier, and feeds back corresponding assay parameter value and parameter identifier to the *in-vitro* diagnostic device through the third-party data service platform, or the manufacturer service platform itself feeds back the corresponding assay parameter value and parameter identifier to the *in-vitro* diagnostic device.

The device, wherein the collection module (200) may comprise:
a reception and recognition module (202) configured to collect the assay parameter identifier by receiving a parameter identifier identification code, wherein the parameter identifier identification code is an identification code input by a user.

In any one of the embodiments of the device, the collection module (200) may further comprise:
a scanning module (204) configured to collect the assay parameter identifier by scanning an identity identification tag, wherein the identity identification tag comprises at least one of one-dimensional barcode, two-dimensional barcode and radio frequency tag.

In any one of the embodiments of the device, the sending module (300) may comprise:
a wireless communication module configured to send the data processing request to the data service platform through wireless communication, wherein the wireless communication comprises 3G, 4G, 5G, WiFi and bluetooth.

### Brief Description of Drawings

In order to more clearly illustrate technical solutions in embodiments of the present disclosure or the prior art, accompanying drawings which are required to be used in the description of the embodiments or the prior art will be introduced briefly below, and apparently, the accompanying drawings in the description below merely show some embodiments of the present disclosure, and those ordinarily skilled in the art still could obtain other accompanying drawings in light of these accompanying drawings, without using creative effort.
FIG. 1 is a flowchart of an *in-vitro* diagnostic device parameter updating method;
FIG. 2 is a flowchart of an *in-vitro* diagnostic device parameter updating method according to another embodiment which is not the claimed invention;
FIG. 3 is a flowchart of an *in-vitro* diagnostic device parameter updating method according to another embodiment which is not the claimed invention;
FIG. 4 is a diagram of application example of an *in-vitro* diagnostic device parameter updating method according to an embodiment;
FIG. 5 is a diagram of application example of an *in-vitro* diagnostic device parameter updating method in another embodiment which is not the claimed invention;
FIG. 6 is a flowchart of an *in-vitro* diagnostic device parameter updating method according to another embodiment which is not the claimed invention;
FIG. 7 is a flowchart of an *in-vitro* diagnostic device parameter updating method according to another embodiment which is not the claimed invention;
FIG. 8 is a structural block diagram of an *in-vitro* diagnostic device parameter updating device according to an embodiment;
FIG. 9 is a structural block diagram of an *in-vitro* diagnostic device parameter updating device according to another embodiment;
FIG. 10 is a structural block diagram of an *in-vitro* diagnostic device parameter updating device according to another embodiment which is not the claimed invention;
FIG. 11 is a structural block diagram of an *in-vitro* diagnostic device parameter updating device according to another embodiment which is not the claimed invention; and
FIG. 12 is an internal structural view of a computer device according to an embodiment which is not the claimed invention.

### Detailed Description of Embodiments

In order to explain the inventive concept and technical solutions of the *in-vitro* diagnostic device parameter updating method and device in the present disclosure in detail, some relevant contents will be described below.

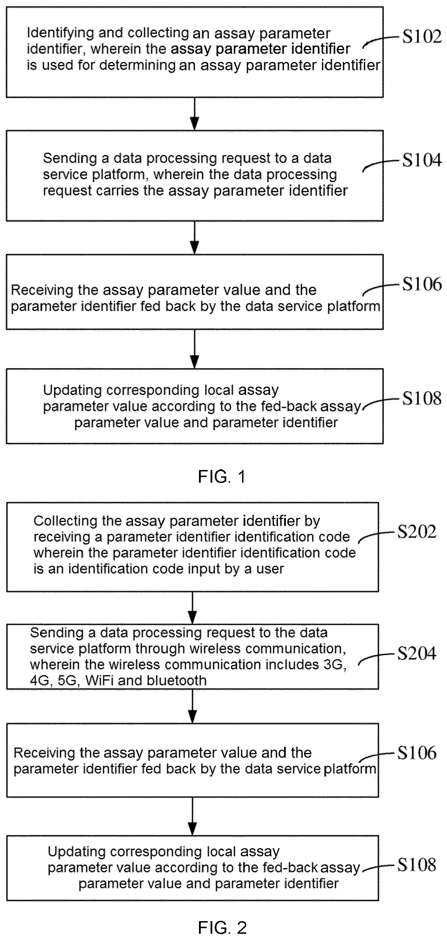

As shown in FIG. 1, an *in-vitro* diagnostic device parameter updating method is provided, including following steps:
S102: identifying and collecting an assay parameter identifier, wherein the assay parameter identifier is used for determining an assay parameter.

In the above, the *in-vitro* diagnostic reagent refers to reagent, reagent kit, calibration product (article), quality control (article) and the like that can be used alone or in combination with an instrument, appliance, device or system for *in-vitro* test of human body samples (various body fluids, cells, tissue samples and so on) during disease prevention, diagnosis, therapy and monitoring, prognostic observation, health state evaluation and genetic disease prediction. The assay refers to a specific analytical item in which the *in-vitro* diagnostic reagent is used for testing and diagnosis, such as TSH (thyrotropin, thyroid stimulating hormone) in thyroid functions, AFP (alpha fetoprotein) in tumors, and HIV (Human Immunodeficiency Virus) in infectious diseases. The assay parameter refers to a parameter associated with the assay of the *in-vitro* diagnostic reagent and is used for determining a reaction process or result of the assay, and the assay parameter generally varies with different assays or different reagent lots. Common parameters include reaction mode (one-step method, two-step method, etc.), sample volume, incubation time, main calibrated curve, calibration data and so on. The assay parameter includes two parts, namely, assay parameter identifier and assay parameter value. The assay parameter identifier refers to an identity identifier or name that can be used to uniquely determine the assay parameter, which may be number, word, picture, symbol, code, etc., and specifically, the parameter identifier may be item information and specific parameter identifier, such as name, production date, lot number, bottle number and validity period of an *in-vitro* diagnostic reagent corresponding to a specific assay, for example, the parameter identifier of incubation time of assay HIV may be HIV_20180207 001 0001 12) .IncuT, HIV_(20180207 001 0001 12). 306, etc.

S104: sending a data processing request to a data service platform, wherein the data processing request carries the assay parameter identifier.

Specifically, the *in-vitro* diagnostic device is configured to send a data processing request to the data service platform, wherein the data processing request carries the assay parameter identifier. After receiving the data processing request, the data service platform determines corresponding assay parameter identifier and assay parameter value according to the assay parameter identifier carried therein, and then feeds back the corresponding assay parameter value and parameter identifier to the *in-vitro* diagnostic device. In the above, the data processing request refers to a request for requesting the data service platform to determine the assay parameter value according to the assay parameter identifier. The data service platform refers to an Internet-based data service platform, which can provide accurate and fast data retrieval and storage, wherein the data service platform is configured to process the assay parameter identifier using data retrieval and search technologies.

S106: receiving the assay parameter value and the parameter identifier fed back by the data service platform.

In the above, the fed-back assay parameter value refer to specific parameter value corresponding to the assay parameter identifier, for example, if a parameter value of incubation time of assay HIV is 10 min (minutes), HIV_(20180207 001 0001 12) .IncuT=10 min can be obtained. Specifically, the data service platform is configured to determine a corresponding assay parameter value according to an assay parameter identifier carried in the data processing request and then to feedback corresponding assay parameter value and parameter identifier to the *in-vitro* diagnostic device.

S108: updating corresponding local assay parameter value according to the fed-back assay parameter value and parameter identifier.

Specifically, the *in-vitro* diagnostic device is configured to update its own corresponding assay parameter value according to the fed-back assay parameter value and parameter identifier. For example, before using an *in-vitro* diagnostic reagent of a new lot number, the parameter of assay of the *in-vitro* diagnostic reagent needs to be recalibrated, wherein first, the assay parameter identifier are identified by the *in-vitro* diagnostic device (at this time, the parameter corresponding to the parameter identifier is main calibrated curve and calibration data), then, a data processing request carrying the assay parameter identifier is sent to the data service platform, then the data service platform performs processing according to the assay parameter identifier, and feeds back the main calibrated curve and the calibration data of the assay to the *in-vitro* diagnostic device, and the *in-vitro* diagnostic device automatically updates the main calibrated curve and the calibration data of its corresponding *in-vitro* diagnostic reagent according to a fed-back result. When a sample of human body is to be detected using the *in-vitro* diagnostic reagent, a detection result will be judged according to the updated main calibrated curve and the updated calibration data, so as to realize precise detection.

In the above *in-vitro* diagnostic device parameter updating method, the assay parameter identifier is transmitted to the data service platform, the assay parameter value and the parameter identifier fed back by the data service platform are received, the corresponding assay parameter value are updated according to the fed-back assay parameter value and parameter identifier, the data is supplied and transmitted through the Internet, then the original method in which barcode, calibration curve card, mobile data storage device and portable device are used as carriers for supplying and transmitting data is changed, the data transmission speed is increased, thereby an efficient updating of assay parameters of an *in-vitro* diagnostic reagent is realized.

In one of the embodiments which is not the claimed invention, as shown in FIG. 2, S102 includes:
S202: collecting the assay parameter identifier by receiving a parameter identifier identification code, wherein the parameter identifier identification code is an identification code input by a user.

Specifically, the *in-vitro* diagnostic device is configured to determine a corresponding assay parameter identifier by receiving the parameter identifier identification code. The parameter identifier identification code may be a set of data consisting of numerals and letters, which may be manually input by the user, and also may be input by the user through voice interaction.

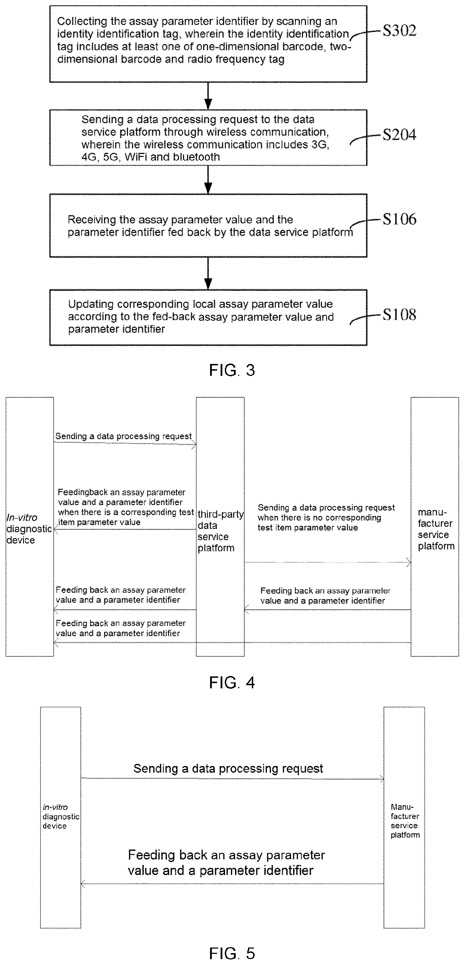

In one of the embodiments which is not the claimed invention, as shown in FIG. 3, S102 includes:
S302: collecting the assay parameter identifier by scanning an identity identification tag, wherein the identity identification tag includes at least one of one-dimensional barcode, two-dimensional barcode and radio frequency tag.

The *in-vitro* diagnostic device is configured to collect the assay parameter identifier by scanning the identity identification tag, and determine the assay parameter of the *in-vitro* diagnostic reagent according to the assay parameter identifier. In the above, the identity identification tag refers to an identifier used for identifying different assay parameter identifier. The one-dimensional barcode refers to a mark composed by a set of regularly arranged bars, blanks and corresponding characters, wherein a "bar" refers to a part with relatively low light reflectance, and a "blank" refers to a part with relatively high light reflectance, and data composed by these bars and blanks expresses certain information, and can be read by a specific device to be converted into binary and decimal information compatible with computer. The two-dimensional barcode (or QR code) refers to a black and white pattern distributed on a plane (in two-dimensional directions) according to a certain rule with a certain specific geometric shape, and is used for recording data symbol information. The radio frequency tag is realized based on radio frequency identification, wherein the radio frequency identification refers to a wireless communication technology by which a specific object can be identified to read/write relevant data through radio signals, without the need of establishing mechanical or optical contact between an identification system and the specific object.

In one of the embodiments which is not the claimed invention, as shown in FIG. 2 and FIG. 3, S104 includes:
S204: sending a data processing request to the data service platform through wireless communication, wherein the wireless communication includes 3G (the 3^{rd} Generation mobile communication technology), 4G (the 4^{th} Generation mobile communication technology), 5G (the 5^{th} Generation mobile communication technology), WiFi (Wireless-Fidelity) and bluetooth.

Specifically, the *in-vitro* diagnostic device is configured to send the data processing request to the data service platform through wireless communication. The wireless communication refers to a communication mode for exchanging information using a property of an electromagnetic wave signal that it can propagate in a free space. 3G refers to a cellular mobile communication technology supporting high-speed data transmission. The 3G service can transmit voice and data information simultaneously, typically at a rate of no less than several hundred of kbps (bit rate). 4G, as an integration of 3G and WLAN (Wireless Local Area Networks), can transmit data, high-quality audios, videos, images and so on at a fast spped. The wireless communication further includes 5G technology, and when realized in a later stage, the 5G technology is also applicable to the present solution.

In one of the embodiments which is not the claimed invention, the assay parameter includes at least one of reaction mode, sampling amount, incubation time, main calibrated curve and calibration data associated with the *in-vitro* diagnostic reagent item.

Specifically, the reaction protocol refers to a reagent reaction process which is determined by the principle of reagent reaction, and for an immune reaction, the reaction protocol generally includes a one-step sandwich method, a two-step competition method, etc. The sampling volume includes sample sampling volume and reagent sampling volume. The sample sampling volume refers to sample volume required in the assay reaction. The reagent sampling volume refers to the reagent volume required in the assay reaction. The incubation time refers to duration of the antigen-antibody binding reaction or biotin-avidin binding reaction of the reactant in a constant temperature environment. The main calibrated curve is a standard curve typically formed by 6-10 concentration points and corresponding signal values established by the manufacturer when manufacturing each new lot of reagents. The calibration is a process in which a user runs 2-3 calibrators with known concentrations, and adjusts the main calibrated curve according to obtained signal values and a corresponding calibration algorithm so as to eliminate difference between instruments of users. The calibration data includes, among other things, quantity and concentration of calibrator, and corresponding calibration algorithm. The assay parameter value refers to a specific parameter value corresponding to the assay parameter, such as 100 microliters of sampling volume, and 10 minutes of incubation time.

In one of the embodiments, the data service platform includes manufacturer service platform and third-party data service platform.

Specifically, when there are too many assay parameters and too large data flow, both the third-party data service platform and the manufacturer service platform are to be used, and when there are not too many assay parameters, it is sufficient to use the manufacturer service platform only. In the above, the manufacturer service platform refers to a server of a manufacturer of the *in-vitro* diagnostic device. The third-party data service platform refers to an Internet-based intelligent data service platform or a self-deployable local data platform provided by a third party.

In one of the embodiments, as shown in FIG. 4, the case in which both the third-party data service platform and the manufacturer service platform are used is illustrated by a data transfer process. The third-party data service platform, as an intermediate server, stores therein partial assay parameter values and parameter identifiers, when the *in-vitro* diagnostic device sends a data processing request, the third-party data service platform first searches itself to find whether a corresponding assay parameter value exists or not, wherein if the corresponding assay parameter value exists, the corresponding assay parameter value and parameter identifier are directly fed back to the *in-vitro* diagnostic device, and if the corresponding assay parameter value does not exist, the data processing request from the *in-vitro* diagnostic device is sent to the manufacturer service platform, the manufacturer service platform determines the corresponding assay parameter value according to the assay parameter identifier, and feeds back corresponding assay parameter value and parameter identifier to the *in-vitro* diagnostic device through the third-party data service platform, or the manufacturer service platform itself feeds back the corresponding assay parameter value and parameter identifier to the *in-vitro* diagnostic device.

In one of the embodiments which is not the claimed invention, as shown in FIG. 5, the case in which only the manufacturer service platform is used is illustrated by a data transfer process. When the *in-vitro* diagnostic device sends the data processing request, the manufacturer service platform determines corresponding assay parameter value according to the assay parameter identifier, and feeds back corresponding assay parameter value and parameter identifier to the *in-vitro* diagnostic device.

It should be understood that although various steps in the flowcharts of FIGS. 1-3 are shown in turn as indicated by arrows, these steps are not necessarily performed in the order as indicated by the arrows. Unless explicitly stated herein, these steps are not limited to being performed in a strict order, while these steps may be performed in other orders. Moreover, at least part of the steps in FIG. 1-3 may include multiple sub-steps or multiple stages that are not necessarily performed simultaneously, but may be performed at different times, and these sub-steps or stages are not necessarily performed in turn, but may be performed with at least a part of other steps or sub-steps of other steps or stages by turns or in an alternating manner.

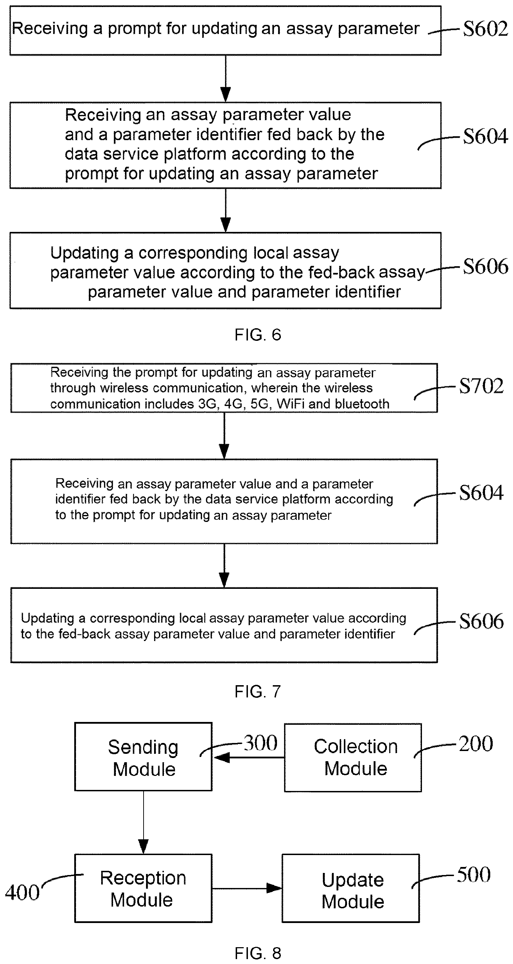

In one of the embodiments which is not the claimed invention, as shown in FIG. 6, an *in-vitro* diagnostic device parameter updating method is provided, including steps of:
S602: receiving a prompt for updating an assay parameter;

Specifically, when considering that an assay parameter needs to be updated, the data service platform actively (voluntarily) sends a prompt for updating the assay parameter to the *in-vitro* diagnostic device.

S604: receiving an assay parameter value and a parameter identifier fed back by the data service platform according to the prompt for updating the assay parameter.

Specifically, after receiving the prompt for updating the assay parameter, the *in-vitro* diagnostic device may select to actively confirm the updating, and then further, the *in-vitro* diagnostic device will receive the assay parameter value and the parameter identifier fed back by the data service platform, so as to perform the updating according to the assay parameter value and the parameter identifier. If the *in-vitro* diagnostic device does not actively confirm the updating after receiving the prompt for updating the assay parameter, the data service platform also feeds back the assay parameter value and the parameter identifier to the *in-vitro* diagnostic device within a preset timing period, for the *in-vitro* diagnostic device to update the assay parameter.

For example, after actively confirming the updating, the *in-vitro* diagnostic device will send an updating confirmation prompt to the data service platform, the data service platform immediately feeds back the assay parameter value and the parameter identifier to the *in-vitro* diagnostic device after receiving the updating confirmation prompt, wherein if no updating confirmation prompt is received within a preset timing period of two days, the assay parameter value and the parameter identifier are also fed back to the *in-vitro* diagnostic device, for the *in-vitro* diagnostic device to update the assay parameter.

S606: updating a corresponding local assay parameter value according to the fed-back assay parameter value and parameter identifier.

Specifically, after receiving the fed-back assay parameter value and parameter identifier, the *in-vitro* diagnostic device updates corresponding local assay parameter value according to the fed-back assay parameter value and parameter identifier.

In one of the embodiments which is not the claimed invention, as shown in FIG. 7, S602 includes:
S702: receiving the prompt for updating the assay parameter through wireless communication, wherein the wireless communication includes 3G, 4G, 5G, WiFi and bluetooth.

In one of the embodiments, as shown in FIG. 8, an *in-vitro* diagnostic device parameter updating device is provided, including: a collection module 200, a sending module 300, a reception module 400 and an update module 500,
wherein the collection module 200 is configured to identify and collect an assay parameter identifier, wherein the assay parameter identifier is used for determining an assay parameter;
the sending module 300 is configured to send a data processing request to a data service platform, wherein the data processing request carries the assay parameter identifier;
the reception module 400 is configured to receive an assay parameter value and a parameter identifier which are fed back by the data service platform; and
the update module 500 is configured to update a corresponding local assay parameter value according to the fed-back assay parameter value and parameter identifier.

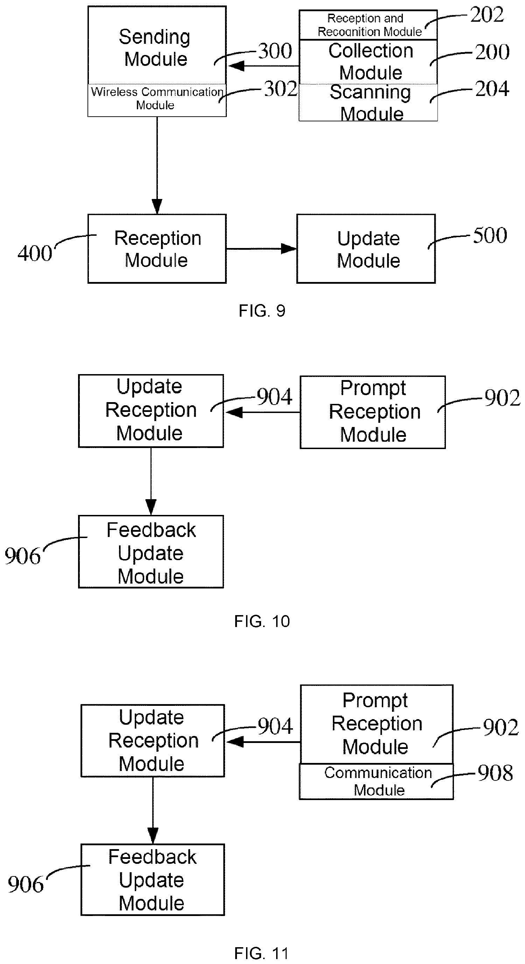

In one of the embodiments, as shown in FIG. 9, the collection module 200 further includes a reception and recognition module 202, configured to collect the assay parameter identifier by receiving a parameter identifier identification code, wherein the parameter identifier identification code is an identification code input by a user.

In one of the embodiments, as shown in FIG. 9, the collection module 200 further includes a scanning module 204, configured to collect the assay parameter identifier by scanning an identity identification tag, wherein the identity identification tag includes at least one of one-dimensional barcode, two-dimensional barcode and radio frequency tag.

In one of the embodiments, as shown in FIG. 9, the sending module 300 further includes a wireless communication module 302, configured to send the data processing request to the data service platform through wireless communication, wherein the wireless communication includes 3G, 4G, 5G, WiFi and bluetooth.

An *in-vitro* diagnostic device parameter updating device which is not the claimed invention, as shown in FIG. 10, includes:
a prompt reception module 902 configured to receive a prompt for updating an assay parameter;
an update reception module 904 configured to receive an assay parameter value and a parameter identifier fed back by the data service platform according to the prompt for updating the assay parameter; and
a feedback update module 906 configured to update a corresponding local assay parameter value according to the fed-back assay parameter value and parameter identifier.

In one of the embodiments, as shown in FIG. 11 which is not the claimed invention, the prompt reception module 902 further includes:
a communication module 908 configured to receive the prompt for updating the assay parameter through wireless communication, wherein the wireless communication includes 3G, 4G, 5G, WiFi and bluetooth.

For specific limitations on the *in-vitro* diagnostic device parameter updating device, reference may be made to definitions to the *in-vitro* diagnostic device parameter updating method in the above, which will not be repeated herein. Various modules in the above *in-vitro* diagnostic device parameter updating device may be wholly or partially implemented by software, hardware and a combination thereof. Various modules above may be embedded in a hardware form in or independent of a processor of the computer device, and may also be stored in a memory of the computer device in a software form, so that the processor can invoke and execute operations corresponding to various modules above.

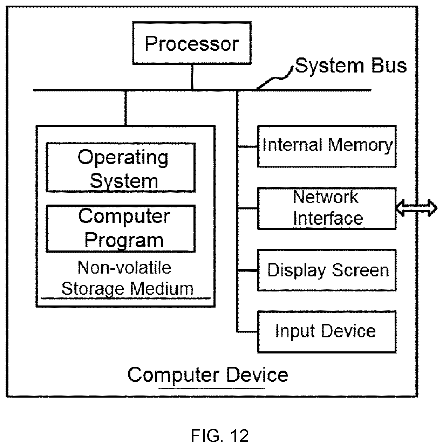

In an embodiment which is not the claimed invention, a computer device is provided, which may be a terminal, and a diagram of internal structure of the computer device may be as shown in FIG. 12. The computer device includes a processor, a memory, a network interface, a display screen and an input device which are connected via a system bus. In the above, the processor of the computer device is configured to provide computing and control capabilities. The memory of the computer device includes a nonvolatile storage medium and an internal memory. An operating system and a computer program are stored on the non-volatile storage medium stores. The internal memory provides an environment for running of the operating system and the computer program in the non-volatile storage medium. The network interface of the computer device is used for communication with an external terminal through network connection. When executed by a processor, the computer program implements an *in-vitro* diagnostic device parameter updating method. A display screen of the computer device may be a liquid crystal display screen or an electronic ink display screen, and an input device of the computer device may be a touch layer covered on the display screen, keys, a track ball or a touch pad arranged on a shell of the computer device, and may also be an external keyboard, a touch pad or a mouse or the like.

It could be appreciated by those skilled in the art that the structure shown in FIG. 12 is merely a block diagram of a portion of the structure associated with the solution of the present disclosure, and does not constitute limitation on the computer device to which the solution of the present disclosure is applied, and that a particular computer device may include more or fewer components than shown in the drawings, or may combine certain components, or have a different arrangement of components.

In an embodiment which is not the claimed invention, a computer device is provided, including a memory and a processor, wherein a computer program is stored in the memory and when executing the computer program, the processor performs following steps:
identifying and collecting an assay parameter identifier, wherein the assay parameter identifier is used for determining an assay parameter;
sending a data processing request to a data service platform, wherein the data processing request carries the assay parameter identifier;
receiving an assay parameter value and a parameter identifier which are fed back by the data service platform; and
updating a corresponding local assay parameter value according to the fed-back assay parameter value and parameter identifier.

In an embodiment which is not the claimed invention, when executing the computer program, the processor further realizes following step:
collecting the assay parameter identifier by receiving a parameter identifier identification code, wherein the parameter identifier identification code is an identification code input by a user.

In an embodiment which is not the claimed invention, when executing the computer program, the processor further realizes following step:
collecting the assay parameter identifier by scanning an identity identification tag, wherein the identity identification tag includes at least one of one-dimensional barcode, two-dimensional barcode and radio frequency tag.

In an embodiment which is not the claimed invention, when executing the computer program, the processor further realizes following step:
sending a data processing request to the data service platform through wireless communication, wherein the wireless communication includes 3G, 4G, 5G, WiFi and bluetooth.

In an embodiment which is not the claimed invention, a computer-readable storage medium is provided, on which a computer program is stored, and when the computer program is executed by a processor, the following steps are realized:
identifying and collecting an assay parameter identifier, wherein the assay parameter identifier is used for determining an assay parameter;
sending a data processing request to a data service platform, wherein the data processing request carries the assay parameter identifier;
receiving an assay parameter value and a parameter identifier which are fed back by the data service platform; and
updating a corresponding local assay parameter value according to the fed-back assay parameter value and parameter identifier.

In an embodiment which is not the claimed invention, when the computer program is executed by the processor, the following step is further realized:
collecting the assay parameter identifier by receiving a parameter identifier identification code, wherein the parameter identifier identification code is an identification code input by a user.

In an embodiment, when the computer program is executed by the processor, the following step is further realized:
collecting the assay parameter identifier by scanning an identity identification tag, wherein the identity identification tag includes at least one of one-dimensional barcode, two-dimensional barcode and radio frequency tag.

In an embodiment which is not the claimed invention, when the computer program is executed by the processor, the following step is further realized:
sending a data processing request to the data service platform through wireless communication, wherein the wireless communication includes 3G, 4G, 5G, WiFi and bluetooth.

Those ordinarily skilled in the art could understand that all or part of the processes of the methods of the above embodiments may be accomplished by relevant hardware instructed by a computer program, wherein the computer program may be stored in a non-volatile computer-readable storage medium, and when the computer program is executed, the processes of the embodiments of various above methods may be included. In the above, any reference to memory, storage, database or other medium used in various embodiments provided in the present disclosure may include non-volatile and/or volatile memory. Non-volatile memory may include read-only memory (ROM), programmable ROM (PROM), electrically programmable ROM (EPROM), electrically erasable programmable ROM (EEPROM) or flash memory. Volatile memory may include random access memory (RAM) or external cache memory. By way of illustration and not limitation, RAM is available in a variety of forms such as static RAM (SRAM), dynamic RAM (DRAM), synchronous DRAM (SDRAM), double data rate SDRAM (DDRSDRAM), enhanced SDRAM (ESDRAM), Synchlink DRAM (SLDRAM), rambus direct RAM (RDRAM), direct rambus dynamic RAM (DRDRAM), and rambus dynamic RAM (RDRAM).

## Claims

1. An *in-vitro* diagnostic device parameter updating method, **characterized by** comprising following steps of:
identifying and collecting an assay parameter identifier (S102), wherein the assay parameter identifier is used for determining an assay parameter;
sending a data processing request to a data service platform (S104), wherein the data processing request carries the assay parameter identifier;
receiving an assay parameter value and the parameter identifier which are fed back by the data service platform (S106); and
updating a corresponding local assay parameter value according to the fed-back assay parameter value and parameter identifier (S108),
wherein the assay parameter comprises reaction mode, sampling amount or incubation time associated with an *in-vitro* diagnostic reagent item,
wherein the data service platform comprises a manufacturer service platform and a third-party data service platform,
wherein the data processing request is sent to the third-party data service platform, then the third-party data service platform searches itself to find whether a corresponding assay parameter value exists or not, and
- if the corresponding assay parameter value exists, the corresponding assay parameter value and parameter identifier are directly fed back to the *in-vitro* diagnostic device, and
- if the corresponding assay parameter value does not exist, the data processing request from the *in-vitro* diagnostic device is sent to the manufacturer service platform, the manufacturer service platform determines the corresponding assay parameter value according to the assay parameter identifier, and feeds back corresponding assay parameter value and parameter identifier to the *in-vitro* diagnostic device through the third-party data service platform, or the manufacturer service platform itself feeds back the corresponding assay parameter value and parameter identifier to the *in-vitro* diagnostic device.

2. The method according to claim 1, wherein the step of identifying and collecting an assay parameter identifier comprises:
collecting the assay parameter identifier by scanning an identity identification tag, wherein the identity identification tag comprises at least one of one-dimensional barcode, two-dimensional barcode and radio frequency tag.

3. The method according to claim 1, wherein the step of identifying and collecting an assay parameter identifier comprises:
collecting the assay parameter identifier by receiving a parameter identifier identification code, wherein the parameter identifier identification code is an identification code input by a user.

4. The method according to any one of claims 1 to 3, wherein the step of sending a data processing request to a data service platform comprises:
sending a data processing request to the data service platform through wireless communication, wherein the wireless communication comprises 3G, 4G, 5G, WiFi and bluetooth.

5. An *in-vitro* diagnostic device parameter updating device, **characterized by** comprising:
a collection module (200) configured to identify and collect an assay parameter identifier, wherein the assay parameter identifier is used for determining an assay parameter;
a sending module (300) configured to send a data processing request to a data service platform, wherein the data processing request carries the assay parameter identifier;
a reception module (400) configured to receive an assay parameter value and the parameter identifier which are fed back by the data service platform; and
an update module (500) configured to update a corresponding local assay parameter value according to the fed-back assay parameter value and parameter identifier,
wherein the assay parameter comprises reaction mode, sampling amount or incubation time associated with an *in-vitro* diagnostic reagent item,
wherein the data service platform comprises a manufacturer service platform and a third-party data service platform, and
wherein the data processing request is sent to the third-party data service platform, then the third-party data service platform searches itself to find whether a corresponding assay parameter value exists or not, and
- if the corresponding assay parameter value exists, the corresponding assay parameter value and parameter identifier are directly fed back to the *in-vitro* diagnostic device, and
- if the corresponding assay parameter value does not exist, the data processing request from the *in-vitro* diagnostic device is sent to the manufacturer service platform, the manufacturer service platform determines the corresponding assay parameter value according to the assay parameter identifier, and feeds back corresponding assay parameter value and parameter identifier to the *in-vitro* diagnostic device through the third-party data service platform, or the manufacturer service platform itself feeds back the corresponding assay parameter value and parameter identifier to the *in-vitro* diagnostic device.

6. The device according to claim 5, wherein the collection module (200) comprises:
a reception and recognition module (202) configured to collect the assay parameter identifier by receiving a parameter identifier identification code, wherein the parameter identifier identification code is an identification code input by a user.

7. The device according to claim 5 or 6, wherein the collection module (200) further comprises:
a scanning module (204) configured to collect the assay parameter identifier by scanning an identity identification tag, wherein the identity identification tag comprises at least one of one-dimensional barcode, two-dimensional barcode and radio frequency tag.

8. The device according to any one of claims 5 to 7, wherein the sending module (300) comprises:
a wireless communication module configured to send the data processing request to the data service platform through wireless communication, wherein the wireless communication comprises 3G, 4G, 5G, WiFi and bluetooth.

## Patentansprüche

1. Verfahren zum Aktualisieren von Parametern einer In-vitro-Diagnosevorrichtung, **dadurch gekennzeichnet, dass** es die folgenden Schritte umfasst:
Identifizieren und Sammeln eines Assay-Parameter-Identifikators (S102), wobei der Assay-Parameter-Identifikator zur Bestimmung eines Assay-Parameters verwendet wird;
Senden einer Datenverarbeitungsanforderung an eine Datendienstplattform (S104), wobei die Datenverarbeitungsanforderung den Assay Parameter-Identifikator trägt;
Empfangen eines Assay-Parameterwerts und eines Parameter-Identifikators, die von der Datendienstplattform zurückgegeben werden; und
Empfangen eines Assay-Parameterwerts und des Parameter-Identifikators, die von der Datendienstplattform zurückgesendet werden (S106), und Aktualisieren eines entsprechenden lokalen Assay-Parameterwerts gemäß dem zurückgesendeten Assay-Parameterwert und Parameter-Identifikators (S108),
Aktualisierung eines entsprechenden lokalen Assay-Parameterwerts entsprechend dem rückgekoppelten Assay-Parameterwert und dem Parameter-Identifikators,
wobei der Assay-Parameter den Reaktionsmodus, die Probenahmemenge oder die Inkubationszeit im Zusammenhang mit einem in-vitro-diagnostischen Reagenzartikel umfasst,
wobei die Datendienstplattform eine Herstellerdienstplattform und eine Datendienstplattform eines Dritten umfasst,
wobei die Datenverarbeitungsanforderung an die Datendienstplattform des Drittanbieters gesendet wird, dann sucht die Datendienstplattform des Drittanbieters selbst, um herauszufinden, ob ein entsprechender Assay-Parameterwert existiert oder nicht, und
- wenn der entsprechende Assay-Parameterwert existiert, der entsprechende Assay-Parameterwert und die Parameter-Identifikator direkt an das In-vitro-Diagnosegerät zurückgegeben werden, und
- Wenn der entsprechende Assay-Parameterwert nicht existiert, wird die Datenverarbeitungsanforderung von dem In-vitro-Diagnosegerät an die Hersteller-Serviceplattform gesendet, die Hersteller-Serviceplattform bestimmt den entsprechenden Assay-Parameterwert entsprechend der Assay-Parameter-Identifikator und gibt den entsprechenden Assay-Parameterwert und die Parameter-Identifikator an das In-vitro-Diagnosegerät über die Daten-Serviceplattform eines Drittanbieters zurück, oder die Hersteller-Serviceplattform selbst gibt den entsprechenden Assay-Parameterwert und die Parameter-Identifikator an das In-vitro-Diagnosegerät zurück.

2. Verfahren nach Anspruch 1, wobei der Schritt des Identifizierens und Sammelns eines Assay-Parameter-Identifikators umfasst: Sammeln des Assay-Parameter-Identifikators durch Scannen eines Identitäts-Identifizierungs-Tags, wobei der Identitäts-Identifizierungs-Tag mindestens einen eindimensionalen Barcode, einen zweidimensionalen Barcode oder einen Radiofrequenz-Tag umfasst.

3. Verfahren nach Anspruch 1, wobei der Schritt des Identifizierens und Sammelns eines Assay-Parameter-Identifikators umfasst: Sammeln des Assay-Parameter-Identifikators durch Empfangen eines Parameter-Identifikator-Identifizierungscodes, wobei der Parameter-Identifikator-Identifizierungscode ein von einem Benutzer eingegebener Identifizierungscode ist.

4. Verfahren nach einem der Ansprüche 1 bis 3, wobei der Schritt des Sendens einer Datenverarbeitungsanforderung an eine Datendienstplattform umfasst: Senden einer Datenverarbeitungsanforderung an die Datendienstplattform über drahtlose Kommunikation, wobei die drahtlose Kommunikation 3G, 4G, 5G, WiFi und Bluetooth umfasst.

5. Vorrichtung zur Aktualisierung der Parameter einer In-vitro-Diagnosevorrichtung, **dadurch gekennzeichnet, dass** sie umfasst:
ein Sammelmodul (200), das so konfiguriert ist, dass es einen Assay-Parameter-Identifikator identifiziert und sammelt, wobei der Assay-Parameter-Identifikator zur Bestimmung eines Assay-Parameters verwendet wird;
ein Sendemodul (300), das so konfiguriert ist, dass es eine Datenverarbeitungsanforderung an eine Datendienstplattform sendet, wobei die Datenverarbeitungsanforderung den Assay Parameter-Identifikator trägt;
ein Empfangsmodul (400), das so konfiguriert ist, dass es einen Assay-Parameterwert und einen Parameter-Identifikator empfängt, die von der Datendienstplattform zurückgegeben werden; und
ein Aktualisierungsmodul (500), das so konfiguriert ist, dass es einen entsprechenden lokalen Assay-Parameterwert entsprechend dem rückgekoppelten Assay-Parameterwert und dem Parameter-Identifikator aktualisiert,
wobei der Assay-Parameter den Reaktionsmodus, die Probenahmemenge oder die Inkubationszeit im Zusammenhang mit einem in-vitro-diagnostischen Reagenzartikel umfasst,
wobei die Datendienstplattform eine Herstellerdienstplattform und eine Datendienstplattform eines Drittanbieters umfasst, und
wobei die Datenverarbeitungsanforderung an die Datenserviceplattform eines Drittanbieters gesendet wird, die Datenserviceplattform des Drittanbieters sich selbst durchsucht, um herauszufinden, ob ein entsprechender Assay-Parameterwert existiert oder nicht, und
- wenn der entsprechende Assay-Parameterwert existiert, der entsprechende Assay-Parameterwert und der Parameter-Identifikator direkt an die In-vitro-Diagnosevorrichtung zurückgegeben werden, und
- wenn der entsprechende Assay-Parameterwert nicht existiert, wenn der entsprechende Assay-Parameterwert nicht existiert, wird die Datenverarbeitungsanforderung von der In-vitro-Diagnosevorrichtung an die Hersteller-Serviceplattform gesendet, die Hersteller-Serviceplattform bestimmt den entsprechenden Assay-Parameterwert gemäß dem Assay-Parameter-Identifikator und gibt den entsprechenden Assay-Parameterwert und den Parameter-Identifikator an die In-vitro-Diagnosevorrichtung über die Daten-Serviceplattform eines Drittanbieters zurück, oder die Hersteller-Serviceplattform selbst gibt den entsprechenden Assay-Parameterwert und den Parameter-Identifikator an die In-vitro-Diagnosevorrichtung zurück.

6. Vorrichtung nach Anspruch 5, wobei das Erfassungsmodul (200) umfasst: ein Empfangs- und Erkennungsmodul (202), das so konfiguriert ist, dass es den Assay-Parameter-Identifikator durch Empfangen eines Parameter-Identifikator-Identifizierungscodes erfasst, wobei der Parameter-Identifikator-Identifizierungscode ein von einem Benutzer eingegebener Identifizierungscode ist.

7. Vorrichtung nach Anspruch 5 oder 6, wobei das Erfassungsmodul (200) fernes Folgendes umfasst: ein Scanmodul (204), das so konfiguriert ist, dass es den Assay Parameter-Identifikator durch Scannen eines Identitätsidentifikations-Tags erfasst, wobei das Identitätsidentifikations-Tag mindestens einen eindimensionalen Barcode, einen zweidimensionalen Barcode oder ein Radiofrequenz-Tag umfasst.

8. Vorrichtung nach einem der Ansprüche 5 bis 7, wobei das Sendemodul (300) umfasst: ein drahtloses Kommunikationsmodul, das so konfiguriert ist, dass es die Datenverarbeitungsanforderung über eine drahtlose Kommunikation an die Datendienstplattform sendet, wobei die drahtlose Kommunikation 3G, 4G, 5G, WiFi und Bluetooth umfasst.

## Revendications

1. Méthode de mise à jour des paramètres d'un dispositif de diagnostic *in vitro,* **caractérisée par** les étapes suivantes:
identifier et collecter un identifiant de paramètre d'essai (S102), l'identifiant de paramètre d'essai étant utilisé pour déterminer un paramètre d'essai ;
envoyer une demande de traitement de données à une plate-forme de services de données (S104), la demande de traitement de données comportant l'identifiant du paramètre d'essai ;
recevoir une valeur de paramètre d'essai et l'identifiant de paramètre qui sont renvoyés par la plate-forme de services de données (S106) ; et
mettre à jour une valeur de paramètre d'essai locale correspondante en fonction de la valeur de paramètre d'essai renvoyée et de l'identifiant de paramètre (S108),
dans laquelle le paramètre d'essai comprend le mode de réaction, la quantité d'échantillonnage ou le temps d'incubation associé à un réactif de diagnostic *in vitro,*
dans laquelle la plate-forme de services de données comprend une plate-forme de services du fabricant et une plate-forme de services de données de tiers,
dans laquelle la demande de traitement des données est envoyée à la plateforme de services de données de tiers, puis la plateforme de services de données de tiers effectue une recherche pour déterminer si une valeur de paramètre d'essai correspondante existe ou non, et
- si la valeur du paramètre d'essai correspondant existe, la valeur du paramètre d'essai correspondant et l'identifiant de paramètre sont directement renvoyés au dispositif de diagnostic *in vitro,* et
- si la valeur du paramètre d'essai correspondant n'existe pas, la demande de traitement de données du dispositif de diagnostic in *vitro* est envoyée à la plate-forme de service du fabricant, la plate-forme de service du fabricant détermine la valeur du paramètre d'essai correspondant en fonction de l'identifiant du paramètre d'essai, et renvoie la valeur du paramètre d'essai correspondant et l'identifiant du paramètre au dispositif de diagnostic *in vitro* par l'intermédiaire de la plate-forme de service de données de tiers, ou la plate-forme de service du fabricant renvoie elle-même la valeur du paramètre d'essai correspondant et l'identifiant du paramètre au dispositif de diagnostic *in vitro.*

2. Méthode selon la revendication 1, dans laquelle l'étape d'identification et de collecte d'un identifiant de paramètre d'essai comprend :
recueillir l'identifiant du paramètre d'essai en scannant une étiquette d'identification, l'étiquette d'identification comprenant au moins un code-barres unidimensionnel, un code-barres bidimensionnel et une étiquette à radiofréquence.

3. La méthode selon la revendication 1, dans laquelle l'étape d'identification et de collecte d'un identifiant de paramètre d'essai comprend :
le recueil de l'identifiant du paramètre d'essai en recevant un code d'identification de l'identifiant du paramètre, le code d'identification de l'identifiant du paramètre étant un code d'identification saisi par un utilisateur.

4. Méthode selon l'une des revendications 1 à 3, dans laquelle l'étape d'envoi d'une demande de traitement de données à une plate-forme de services de données comprend :
l'envoi d'une demande de traitement de données à la plate-forme de services de données par le biais d'une communication sans fil, la communication sans fil comprenant la 3G, la 4G, la 5G, le WiFi et le Bluetooth.

5. Dispositif de mise à jour des paramètres d'un dispositif de diagnostic in *vitro,* **caractérisé par le fait qu'**il comprend:
un module de collecte (200) configuré pour identifier et collecter un identifiant de paramètre d'essai, l'identifiant de paramètre d'essai étant utilisé pour déterminer un paramètre d'essai ;
un module d'envoi (300) configuré pour envoyer une demande de traitement de données à une plateforme de services de données, la demande de traitement de données comportant l'identifiant du paramètre d'essai ;
un module de réception (400) configuré pour recevoir une valeur de paramètre d'essai et l'identifiant de paramètre qui sont renvoyés par la plate-forme de service de données ; et
un module de mise à jour (500) configuré pour mettre à jour une valeur de paramètre d'essai locale correspondante en fonction de la valeur de paramètre d'essai et de l'identifiant de paramètre renvoyés,
dans lequel le paramètre d'essai comprend le mode de réaction, la quantité d'échantillonnage ou le temps d'incubation associé à un réactif de diagnostic *in vitro,*
dans lequel la plate-forme de services de données comprend une plate-forme de services du fabricant et une plate-forme de services de données de tiers, et
dans lequel la demande de traitement des données est envoyée à la plateforme de services de données de tiers, puis la plateforme de services de données de tiers effectue une recherche pour déterminer si une valeur de paramètre d'essai correspondante existe ou non, et
- si la valeur du paramètre d'essai correspondant existe, la valeur du paramètre d'essai correspondant et l'identifiant de paramètre sont directement renvoyés au dispositif de diagnostic *in vitro,* et
- si la valeur du paramètre d'essai correspondant n'existe pas, la demande de traitement de données du dispositif de diagnostic *in vitro* est envoyée à la plate-forme de service du fabricant, la plate-forme de service du fabricant détermine la valeur du paramètre d'essai correspondant en fonction de l'identifiant du paramètre d'essai, et renvoie la valeur du paramètre d'essai correspondant et l'identifiant du paramètre au dispositif de diagnostic *in vitro* par l'intermédiaire de la plate-forme de service de données de tiers, ou la plate-forme de service du fabricant renvoie elle-même la valeur du paramètre d'essai correspondant et l'identifiant du paramètre au dispositif de diagnostic in *vitro.*

6. Dispositif selon la revendication 5, dans lequel le module de collecte (200) comprend :
un module de réception et de reconnaissance (202) configuré pour collecter l'identifiant du paramètre d'essai en recevant un code d'identification de l'identifiant du paramètre, le code d'identification de l'identifiant du paramètre étant un code d'identification saisi par un utilisateur.

7. Dispositif selon la revendication 5 ou 6, dans lequel le module de collecte (200) comprend en outre :
un module de lecture (204) configuré pour collecter l'identifiant du paramètre d'essai en lisant une étiquette d'identification, l'étiquette d'identification comprenant au moins un code-barres unidimensionnel, un code-barres bidimensionnel et une étiquette à radiofréquence.

8. Dispositif selon l'une quelconque des revendications 5 à 7, dans lequel le module d'envoi (300) comprend:
un module de communication sans fil configuré pour envoyer la demande de traitement de données à la plate-forme de services de données par le biais d'une communication sans fil, la communication sans fil comprenant la 3G, la 4G, la 5G, le WiFi et le Bluetooth.
